# EUROPEAN PATENT APPLICATION

(11) **EP 4 364 753 A1**
(43) Date of publication of application: **08.05.2024**
(21) Application number: 22831085.0
(22) Date of filing: 28.06.2022
(51) Int. Cl.: A61K 39/00, A61K 39/39, C07K 14/00, A61P 33/10

(54) **VETERINARY VACCINE COMPOSITION AGAINST PARASITIC WORMS, METHOD FOR TREATING AND PREVENTING INFECTION BY PARASITIC WORMS, AND USE**

(30) Priority: 29.06.2021 BR 102021012953
(71) Applicant: FABP BIOTECH DESENVOLVIMENTO EM BIOTECNOLOGIA LTDA., 22611-100 Rio de Janeiro, RJ (BR); Fundação Oswaldo Cruz - FIOCRUZ, 21045-900 Rio de Janeiro RJ (BR)
(72) Inventor: TENDLER, Miriam, 22611-100 Rio de Janeiro, RJ (BR); RAMOS, Celso, Raúl, Romero, Jardim Esmeralda São Paulo, SP (BR); SOUSA, Gabriel, Limaverde, Soares, Costa, 22460-050 Rio de Janeiro, RJ (BR)
(74) Representative: Meissner Bolte Partnerschaft mbB
(86) International application number: PCT/BR2022/050243
(87) International publication number: WO 2023/272370

(57) **Abstract**

The present invention refers to a veterinary vaccine composition based on Fatty Acid Binding Proteins (FABPs) from parasites. Particularly, the invention presents a veterinary vaccine composition based on *Schistosoma mansoni* rSm14 protein or *Fasciola hepatica* homologous proteins (FhFABPs) which provide a homogeneous and long-lasting immune response against helminths. The invention also aims to provide a method for the treatment and the prevention of infection caused by helminths, particularly *Fasciola hepatica,* as well as the use of these proteins in a vaccine composition against helminths.

## Description

### FIELD OF THE INVENTION

The present invention refers to a veterinary vaccine composition based on Fatty Acid Binding Proteins (FABPs) from parasites. Particularly, the invention presents a veterinary vaccine composition based on *Schistosoma mansoni* rSm14 protein or or *Fasciola hepatica* homologous proteins (FhFABPs) which provide a homogeneous and long-lasting immune response against helminths.

The invention also aims to provide a method for the treatment and the prevention of infection caused by helminths, particularly *Fasciola hepatica,* as well as the use of these proteins in a vaccine composition against helminths.

### BACKGROUND OF THE INVENTION

Vaccines are used to prevent diseases in animals. The effectiveness of a vaccine depends on many variables, such as the nature and quantity of the antigen administered and, especially, the presence of adjuvants to increase immunogenicity.

Among helminths, digenetic trematodes comprise more than 100 families. Most of them are relatively non-aggressive parasites that live in the intestines and other organs of vertebrates and, consequently, have received little attention from parasitologists who make use of applied parasitology. Those trematodes that cause serious disease in humans are the bloodstream trematodes *Schistosoma* and the trematodes of the liver and the lung which are particularly important parasites that infect animals.

*Fasciola,* which is the most important trematode of the liver, is the preferred parasite in domestic ruminants and is responsible for serious economic losses worldwide as it affects cattle, sheep, and goats.

The main characteristic of the disease, and which is responsible for the pathology, morbidity and mortality of the animals mentioned, is based on the destruction of the host's liver tissue as a consequence of the damage caused to the bile ducts where the adult *Fasciola* specimen lives. Morbidity is high in young animals that are especially infected by *Fasciola hepatica* and die as a result. *Fasciola* can sometimes also parasitize humans, when they come into contact with the habitat of the animal disease, and this is most common in Cuba and some Latin American countries. However, the real trematode of the human liver is another parasite, called *Clonorchis sinensis,* which is widespread in China, Japan, Korea, Vietnam, and India. The pathology is basically caused by thickening of the walls of the bile ducts and, in more severe cases, causes cirrhosis of the liver and death.

Both *Fasciola* and *Clonorchis* enter the host passively as a larval form called metacercaria ingested with food (pasture and raw fish for *Fasciola* and *Clonorchis,* respectively), but their migration route in the vertebrate host's organism is through the bile ducts and they differ from each other. While *Clonorchis* gains the biliary tree through the intestine and via the ampulla of Vater, *Fasciola* migrates through the abdominal cavity actively penetrating the liver wall through its capsule, gaining the parenchyma and then the biliary system causing severe damage to the host's tissues.

*Fasciola gigantica* is another trematode so closely related to *F*. *hepatica* in terms of genetics, behavior, and morphological and anatomical structures that it is notoriously difficult to tell them apart. These species of worms infect the liver of various mammals, including humans. Fasciolosis is one of the most important diseases of ruminant animals and affects millions of people, inducing chronic liver pathologies.

The development of new efficient and safe vaccine compositions that include adjuvants for vaccination purposes that support the induction and maintenance of an immune response represents a challenging problem.

Adjuvants are generally defined as compounds that can increase and/or modulate the intrinsic immunogenicity of an antigen. Adjuvants are therefore needed to help new vaccines induce potent and persistent immune responses. In addition, adjuvants need to offer advantages, including more heterologous antibody responses, covering pathogen diversity, induction of potent functional antibody responses, ensuring killing or neutralization of pathogens and induction of more effective T-cell responses, to direct and indirect pathogens, particularly the induction of cytotoxic T cells that are part of a Th1 immune response.

In addition, there are also economic losses from the disease. Khalid Mehmood et al., in "A review on epidemiology, global prevalence and economical losses of fasciolosis in ruminants." Microbial Pathogenesis, Vol. 109, August 2017: 253 - 262, presents a study of this neglected disease found in more than 50 countries, especially where sheep and cattle are raised. Fasciolosis causes significant financial losses due to decreased production and condemnation of animal viscera. Khalid Mehmood et al provide a comprehensive overview of the prevalence and epidemiology of fasciolosis in farm animals from a global perspective. The financial liability related to fasciolosis in livestock production was also addressed.

Ishmael Festus Jajaa et al. in "Financial loss estimation of bovine fasciolosis in slaughtered cattle in South Africa", Parasite Epidemiology and Control. 2017 Oct 12; 2(4): 27 - 34., evaluated the prevalence and monetary losses associated with Fasciola infection in three slaughterhouses in South Africa. Among the data on disease prevalence, the article shows that the total economic loss due to Fasciola infection during the active survey of the three slaughterhouses was USD 3456.2. In addition, the article shows that total liver condemnation was USD 2357 and partial liver condemnation was USD 1515.4.

Verónica Molina-Hernández et al. in "Fasciola hepatica vaccine: We may not be there yet but we're on the right road", Veterinary Parasitology, Vol. 208, Issues 1 - 2. 28 Feb 2015: 101 - 11, points out that great strides have been made in identifying potential vaccine molecules for the control of fasciolosis in cattle, but the level of efficacy required for commercialization has not yet been reached. It is also mentioned in the article that future efforts need to focus on understanding how the parasites invade and penetrate the tissues of their hosts and how they potentiate and control the resulting immune responses, especially in the first few days of infection.

The most pertinent state of the art known to the inventors is patent BR102012026503, granted to OURO FINO SAUDE ANIMAL PARTICIPAÇÕES S.A., which presents a pharmaceutical formulation comprising an aqueous phase and an oil phase.

The aqueous phase contains:
(a) 30 - 40 % of antigens from the virus that causes foot-and-mouth disease;
(b) 0 to 10 % of aqueous vehicle (preferred: saline solution); and,
(c) 0 - 1 % of saponin adjuvant.

The oil phase contains:
(d) 50 - 70 % of oil vehicle (preferred: mineral oil); and,
(e) 0,01 - 2 % of dimethicone-derived polymeric surfactant.

Formulation 3 of document BR102012026503 contains: "a mixture of mineral oil plus Cetyl PEG/PPG-1 0 /1 Dimethicone", in a proportion of 40 % water with 60 % oil plus surfactant. Frmulations 1 and 2 were made with other ingredients for comparison purposes with formulation 3 which represents the invention described therein. However, despite the fact that document BR102012026503 mentions that its composition contains 0 - 1 % of saponin adjuvant, formulation 3 does not contain saponin.

The mention of saponin only appears in the claims of patent BR102012026503, for this reason we cannot state that a person skilled in the art can understand and make the invented object or even use the teachings described in the aforementioned document and distinguish the invention described therein from the state of the art.

Thus, there remains a need to develop an effective vaccine against helminths, especially Fasciolosis, which contains adjuvants that, among other safety aspects, induce potent and persistent immune responses.

### SUMMARY OF THE INVENTION

The present invention provides a veterinary vaccine composition based on Fatty Acid Binding Proteins (FABPs) from parasites. Particularly, the invention presents a veterinary vaccine composition based on *Schistosoma mansoni* rSm14 protein or *Fasciola hepatica* homologous proteins (FhFABPs) which provides a homogeneous and long-lasting immune response against helminths.

The invention also aims to provide a method for the treatment and the prevention of infection caused by helminths, in particular, *Fasciola hepatica.*

### BRIEF DESCRIPTION OF THE FIGURES

Figure 1 shows the titration of total IgG antibodies in individual sera from animals immunized with the different formulations.
Figure 2 shows the titration of IgG1 and IgG2 antibodies in individual sera from animals immunized with the different formulations.
Figure 3 shows the average ELISA readings of anti-rSm14 IgG antibodies in individual sera from the experiment comparing oil formulations in sheep (dilution 1/2000).
Figure 4 shows the titration of the pools of sera from the experimental groups for anti-Sm14 IgG antibodies.
Figure 5 shows the Western Blotting analysis of the recognition of the Sm14 and FhFABP-3 proteins from *Fasciola hepatica* by the pool of sera from animals immunized with Formulation B after 42 days (1/10,000 dilution).
Figures 6A and 6B show the average ELISA readings of anti-Sm14 IgG antibodies in individual sera from cattle immunized with 80 ug (Fig. 6A) and 160 ug (Fig. 6B) of the rSm14 antigen (dilution 1/800).
Figure 7 shows the SDS-PAGE and Western Blot analysis of the rSm14 protein from Formulation B after 20 months of storage at room temperature (22 °C).
Figure 8 shows the rSm14 protein analysis in Formulation B stored for one month at different temperatures.
Figure 9 shows the titration of pools of sera for anti-Sm14 IgG antibodies at 42 days from two immunization experiments conducted with the same batch of vaccine, one year apart.
Figures 10A, 10B and 10C show models of Sm14, FhFABP-3 and FhFABP-V proteins, structural alignment between them and percentage of identity between the sequences and RMSD between the structures of Sm14 and the two FABPs from *Fasciola hepatica.*

### DETAILED DESCRIPTION OF THE INVENTION

The antigen used in the veterinary vaccine composition described herein contemplates, in a very preferential embodiment, the rSm14 protein from *Schistosoma mansoni* or FhFABP-3 and FhFABP-V proteins, the latter two from *Fasciola hepatica,* which are homologous to the rSm14 protein.

The veterinary vaccine formulation of the present invention uses the rSm14 protein as an antigen, obtained in accordance with the Brazilian patents PP1100551, PI0303266 and PI1005855**,** and the patent application BR102017001309**,** incorporated herein by reference.

The Brazilian patent PP1100551 **(DOC01 is equal to** US5730984**),** granted to one of the inventors named herein, provides an antigen to confer protective immunity against helminthic infections. The antigen of the aforementioned patent is a recombinant protein with protective activity against infections caused by pathogenic helminths in humans and animals. Particularly, the **patent PP1100551** teaches the obtaining of the recombinant form of Sm14, rSm14, which is a fusion protein with the capsid protein of bacteriophage T7. The rSm14 protein contains a polypeptide chain consisting of all or part of the amino acid sequence of SEQ ID NO: 1, shown below, and the protective antigenicity is determined by the formation of discontinuous epitopes that are located mainly in the C-terminal portion of Sm14.
SEQ ID NO: 1 - corresponds to the Sm14M20C62 protein
Met Ser Ser Phe Leu Gly Lys Trp Lys Leu Ser Glu Ser His Asn Phe Asp Ala Val Met Ser Lys Leu Gly Val Ser Trp Ala Thr Arg Gln Ile Gly Asn Thr Val Thr Pro Thr Val Thr Phe Thr Met Asp Gly Asp Lys Met Thr Met Leu Thr Glu Ser Thr Phe Lys Asn Leu Ser Cys Thr Phe Lys Phe Gly Glu Glu Phe Asp Glu Lys Thr Ser Asp Gly Arg Asn Val Lys Ser Val Val Glu Lys Asn Ser Glu Ser Lys Leu Thr Gln Thr Gln Val Asp Pro Lys Asn Thr Thr Val Ile Val Arg Glu Val Asp Gly Asp Thr Met Lys Thr Thr Val Thr Val Gly Asp Val Thr Ala Ile Arg Asn Tyr Lys Arg Leu Ser

The Brazilian patent PI0303266 **(DOC02, including DOC02-sequences),** granted to one of the inventors named herein, provides new mutant forms of the Sm14 protein for the production of larger volumes of said protein. The recombinant Sm14 proteins (rSm14), obtained according to patent PI0303266, are defined by the sequences SEQ ID NO: 2, SEQ ID NO: 3, SEQ ID NO: 4, and SEQ ID NO: 5. The mentioned sequences are presented below.
**SEQ ID NO: 2** - corresponds to the Sm14T20C62 protein
   Met Ser Ser Phe Leu Gly Lys Trp Lys Leu Ser Glu Ser His Asn Phe Asp Ala Val Thr Ser Lys Leu Gly Val Ser Trp Ala Thr Arg Gln Ile Gly Asn Thr Val Thr Pro Thr Val Thr Phe Thr Met Asp Gly Asp Lys Met Thr Met Leu Thr Glu Ser Thr Phe Lys Asn Leu Ser Cys Thr Phe Lys Phe Gly Glu Glu Phe Asp Glu Lys Thr Ser Asp Gly Arg Asn Val Lys Ser Val Val Glu Lys Asn Ser Glu Ser Lys Leu Thr Gln Thr Gln Val Asp Pro Lys Asn Thr Thr Val Ile Val Arg Glu Val Asp Gly Asp Thr Met Lys Thr Thr Val Thr Val Gly Asp Val Thr Ala Ile Arg Asn Tyr Lys Arg Leu Ser
**SEQ ID NO: 3** - corresponds to the Sm14A20C62 protein
   Met Ser Ser Phe Leu Gly Lys Trp Lys Leu Ser Glu Ser His Asn Phe Asp Ala Val Ala Ser Lys Leu Gly Val Ser Trp Ala Thr Arg Gln Ile Gly Asn Thr Val Thr Pro Thr Val Thr Phe Thr Met Asp Gly Asp Lys Met Thr Met Leu Thr Glu Ser Thr Phe Lys Asn Leu Ser Cys Thr Phe Lys Phe Gly Glu Glu Phe Asp Glu Lys Thr Ser Asp Gly Arg Asn Val Lys Ser Val Val Glu Lys Asn Ser Glu Ser Lys Leu Thr Gln Thr Gln Val Asp Pro Lys Asn Thr Thr Val Ile Val Arg Glu Val Asp Gly Asp Thr Met Lys Thr Thr Val Thr Val Gly Asp Val Thr Ala Ile Arg Asn Tyr Lys Arg Leu Ser
**SEQ ID NO: 4** - corresponds to the Sm14M20S62 protein
   Met Ser Ser Phe Leu Gly Lys Trp Lys Leu Ser Glu Ser His Asn Phe Asp Ala Val Met Ser Lys Leu Gly Val Ser Trp Ala Thr Arg Gln Ile Gly Asn Thr Val Thr Pro Thr Val Thr Phe Thr Met Asp Gly Asp Lys Met Thr Met Leu Thr Glu Ser Thr Phe Lys Asn Leu Ser Ser Thr Phe Lys Phe Gly Glu Glu Phe Asp Glu Lys Thr Ser Asp Gly Arg Asn Val Lys Ser Val Val Glu Lys Asn Ser Glu Ser Lys Leu Thr Gln Thr Gln Val Asp Pro Lys Asn Thr Thr Val Ile Val Arg Glu Val Asp Gly Asp Thr Met Lys Thr Thr Val Thr Val Gly Asp Val Thr Ala Ile Arg Asn Tyr Lys Arg Leu Ser
**SEQ ID NO: 5** - Sm14M20V62 (PI1005855)
   Met Ser Ser Phe Leu Gly Lys Trp Lys Leu Ser Glu Ser His Asn Phe Asp Ala Val Met Ser Lys Leu Gly Val Ser Trp Ala Thr Arg Gln Ile Gly Asn Thr Val Thr Pro Thr Val Thr Phe Thr Met Asp Gly Asp Lys Met Thr Met Leu Thr Glu Ser Thr Phe Lys Asn Leu Ser Val Thr Phe Lys Phe Gly Glu Glu Phe Asp Glu Lys Thr Ser Asp Gly Arg Asn Val Lys Ser Val Val Glu Lys Asn Ser Glu Ser Lys Leu Thr Gln Thr Gln Val Asp Pro Lys Asn Thr Thr Val Ile Val Arg Glu Val Asp Gly Asp Thr Met Lys Thr Thr Val Thr Val Gly Asp Val Thr Ala Ile Arg Asn Tyr Lys Arg Leu Ser
**SEQ ID NO: 6** - FhFABP-3 protein - Fatty acid-binding protein type 3 - *Fasciola hepatica* (GenBank Q9U1G6.1). SEQ ID NO: 6 has the following sequence:
   Met Ala Asn Phe Val Gly Ser Trp Lys Leu Glu Gln Ser Glu Asn Met Asp Ala Val Leu Gln Lys Leu Gly Ile Asn Val Ile Lys Arg Lys Leu Ile Thr Ser Ser Lys Pro Glu Ile Thr Phe Thr Leu Glu Gly Asn Lys Met Thr Met Lys Thr Val Ser Ala Leu Lys Thr Thr Val Ile Ser Phe Thr Phe Gly Glu Glu Phe Lys Glu Glu Thr Ala Asp Gly Arg Thr Val Met Thr Thr Phe Thr Lys Asp Ser Asp Ser Lys Ile Ser Gln Val Gln Lys Cys Pro Glu Asn Thr Thr His Val Val Arg Glu Val Thr Gly Gly Lys Met Ile Ala Thr Val Thr Val Gly Asp Val Lys Ala Val Asn Asn Tyr His Lys Val
**SEQ ID NO: 7** - FhFABP-V protein - Fatty acid-binding protein type V - *Fasciola hepatica* (GenBank AJO53793.1). A SEQ ID NO: 7 has the following sequence: Met Ser Gly Phe Ile Gly Lys Trp Lys Leu Val Asp Ser Arg Asp Phe Asp Lys Val Met Val Glu Leu Gly Val Gly Tyr Met Thr Arg Lys Ile Ala Glu Asn Thr Lys Pro Thr Val Thr Ile Thr Lys Phe Gly Glu Asp Gly Leu Thr Met Lys Thr Glu Ser Thr Phe Lys Thr Ser Glu Ile Ser Phe Gln Phe Gly Val Glu Phe Asp Glu Thr Thr Ala Asp Gly Arg Gln Val Lys Ser Thr Val Thr Lys Asp Ser Asp Tyr Arg Ile Thr Gln Val Gln Lys His Pro Asn Ala Asp Thr His Ile Val Arg Gln Val Glu Asn Asp Ile Met Asp Thr Thr Val Thr Val Arg Asp Val Val Ser His Arg Arg Tyr Gln Arg Ile Lys

The present invention can use any of the proteins defined by SEQ ID NOS:1 to 7.

Sequences SEQ ID NO: 6 and SEQ ID NO: 7 correspond to the FABPs from *Fasciola hepatica* with the highest homology with Sm14 (Morphew RM, et al. Exploring and Expanding the Fatty-Acid-Binding Protein Superfamily in Fasciola Species. J Proteome Res. 2016. 15(9): 3308 - 21).

The process for purifying the proteins used in the invention is the one described in the Brazilian patent BRPI1005855 (corresponding to US patent 9.475.838 B2), which is briefly incorporated here for reference purposes.

The proteins initially used in the present invention are those defined by SEQ ID NO: 5, SEQ ID NO: 6 and SEQ ID NO: 7 and were obtained from a genetically manipulated strain of *Pichia pastoris,* according to the teachings of the Brazilian patent BRPI1005855. The purification process for the recombinant protein expressed in *Pichia pastoris* comprises the following steps:
(a) lysing the *P. pastoris* cells
(b) clarifying the lysate obtained in the step (a)
(c) loading the clarified lysate onto anion exchange resin and after loading the protein, the protein is eluted by pH exchange in the column
(d) separating contaminating proteins from the recombinant protein by gel-filtration.

These proteins have the potential to be used as antigens for a specific formulation for *Fasciola hepatica.* Sera from animals immunized with Sm14 recognize the FABP type 3 protein from *Fasciola hepatica.*

The Brazilian patent PI1005855, teaches the process of producing recombinant Sm14 protein in *Pichia pastoris,* to be used in the present invention, which consists of:
(a) synthesizing the gene defined by SEQ ID NO: 5 mentioned above;
(b) cloning the gene synthesized in step (a) into the pPIC9K vector, through the BamHI site and reconstituting the Kozak sequence of the AOXI gene before the Sm14 protein start codon, for intracellular Sm14 protein expression; and,
(c) transforming the *P. pastoris* with the plasmid pPIC9K-Sm14-MV and selection of recombinant clones with multiple copies.

The production process developed for the Sm 14 protein can be used for the recombinant production of homologous proteins from *Fasciola hepatica,* such as FABP type 3 and type V (SEQ ID NO: 07 and 08), which have physicochemical characteristics similar to the Sm14 protein (in terms of molecular mass and isoelectric point) (pI)).

The production process is described in the Brazilian patent application BR102017001309, incorporated herein for reference purposes.

Brazilian patent application BR102017001309 provides a process for producing proteins in recombinant form using a synthetic gene for high protein expression in *Pichia pastoris.* More specifically, the invention describes the production of the Sm14 protein from *Schistosoma mansoni* in recombinant form, where a synthetic gene for high expression of this protein was created, which was cloned under the control of two types of *Pichia pastoris* promoters: methanol-inducible promoter (AOX1) and constitutive promoter (GAP). With these constructs, *Pichia pastoris* strains were genetically manipulated to efficiently produce the Sm14 vaccine antigen. Processes to produce and purify this protein from *P. pastoris* cells have also been perfected and can be scaled up for industrial production. The protein of interest is the protein with the pGAP-9k/Sm 14-M20V62 construct, corresponding to the SEQ ID NO: 5.

Thus, based on all the knowledge gained from the behavior of the rSm14 protein, but without being limited by theory, the present inventors have developed, in the preferred embodiment of the invention, a vaccine composition containing the rSm14 protein or FhFABP-3 or FhFABP-V, defined by SEQ ID NO: 5, 6 and 7 in a water-in-oil emulsion (W/O).

The veterinary vaccine composition of the present invention comprises the antigen and adjuvants.

Throughout the present description, the term saponin comprises glycosides and surfactants that can be obtained from plants, with the most effective saponins as adjuvants being those obtained from the South American tree *Quillaja saponaria,* preferably from the bark of that tree. During the isolation of saponins from *Quillaja saponaria,* an extract is obtained which in partially purified form is known as "Quil A", also included within the scope of the invention.

Among the adjuvants selected for the present invention, the W/O emulsion of the invention comprises adjuvants such as saponin or Quil-A, Tris-HCl buffer, Thimerosal, and water. The oil phase comprises at least one of Marcol52^{™} oil, Cetyl PEG/PPG-10/1 Dimethicone, Span 80 or Tween 80.

In the following the invention will be described in its most preferred way.

The amount of antigen used in the composition can vary within the range of 50 ug to 200 ug, preferably 80 ug for sheep and 160 for cattle.

More specifically, the composition of the invention for a dose volume of 2 ml, where the aqueous phase corresponds to 40 % of the dose (0.8 ml) and the oil phase corresponds to 60 % of the dose (1.2 ml).

**TABLE A**

| Ingredient | Amount |
|---|---|
| Aqueous phase | |
| Antigen (rSm14, FhFABP-3 or FhFABP-V) | from 50 to 200 ug |
| Saponin | from 0.6 to 1 mg |
| Thimerosal | 0.01 % |
| Tris-HCl Buffer 50mM, q.s. | 0.8 ml |

| Oil phase | |
|---|---|
| Marcol 52^{™} oil | 1.2 ml |
| Cetyl PEG/PPG-10/1 Dimethicone | 20 mg |

The protein was purified according to the steps below, which are described in the Brazilian patent BRPI1005855.
(a) lysing the *P. pastoris* cells
(b) clarifying the lysate obtained in the step (a)
(c) loading the clarified lysate onto anion exchange resin and after loading the protein, the protein is eluted by pH exchange in the column
(d) separating contaminating proteins from the recombinant protein by gel-filtration.

The water-in-oil emulsion, in a volume of 1 liter, was prepared under aseptic conditions. Initially, the aqueous and oil phases were mixed in a beaker with a magnetic stirrer at room temperature (around 22 °C) for 30 minutes (pre-homogenization). The mixture is then passed three times through a high-speed Silverson homogenizer at 9000 rpm, keeping the temperature between 15 °C and 37 °C.

After describing the invention with reference to certain preferred embodiments, other embodiments will become clear to a person skilled in the art. The invention is further defined by reference to the following examples, describing in detail the preparation of the composition and methods for the treatment and prevention of the disease. It will be clear that many modifications, both to materials and methods, can be practiced without departing from the scope of the invention.

### EXAMPLES

The following examples are intended to show the results of adjuvant research, comparison of oil formulations, dose response in cattle and sheep, and formulation stability.

### EXAMPLE 1: Comparison of the immunogenicity of different formulations of the Sm14 protein

The purpose of the following Example is to evaluate different formulations of the Sm14 protein in order to select the one that performs best in terms of stimulating the immune response.

Groups of 6 adult sheep were immunized with the following formulas:

**Table 1: Selection of vaccine formulations containing the Sm14 antigen**

| **Group** | **Composition** | **Type of formulation** |
|---|---|---|
| **1** | Placebo (saline solution) | Aqueous solution |
| **2** | Antigen | Aqueous solution |
| **3** | Antigen + 2 % of mineral oil | O/A |
| **4** | Antigen + 2 % of mineral oil + imiquimod | O/A |
| **5** | Antigen + 2 % of mineral oil + imiquimod + Quil-A | O/A |
| **6** | Antigen + 2 % of mineral oil + imiquimod + MDP | O/A |
| **7** | Antigen + 60 % of mineral oil + saponin | A/O |

Group 2, immunized only with the Sm14 antigen in an aqueous solution, served as a control formulation. The immunization of Group 3 shows the effect of mineral oil in stimulating the immune response, in an oil-in-water (O/W) formulation. Group 4 shows the effect of adding Imiquimod, a Toll Like Receptor 7 (TLR-7) agonist, to the oil formulation. Groups 5 and 6 allowed us to study the synergism of the addition of Quil-A (saponin-derived fraction) and MDP (Muramil Dipeptide, NOD receptor agonist). Group 7 was immunized with a water-in-oil formulation containing saponin.

The animals were immunized at 0 and 28 days. Serum samples were collected every 14 days, until 98 days after the first dose, to assess the immune response.

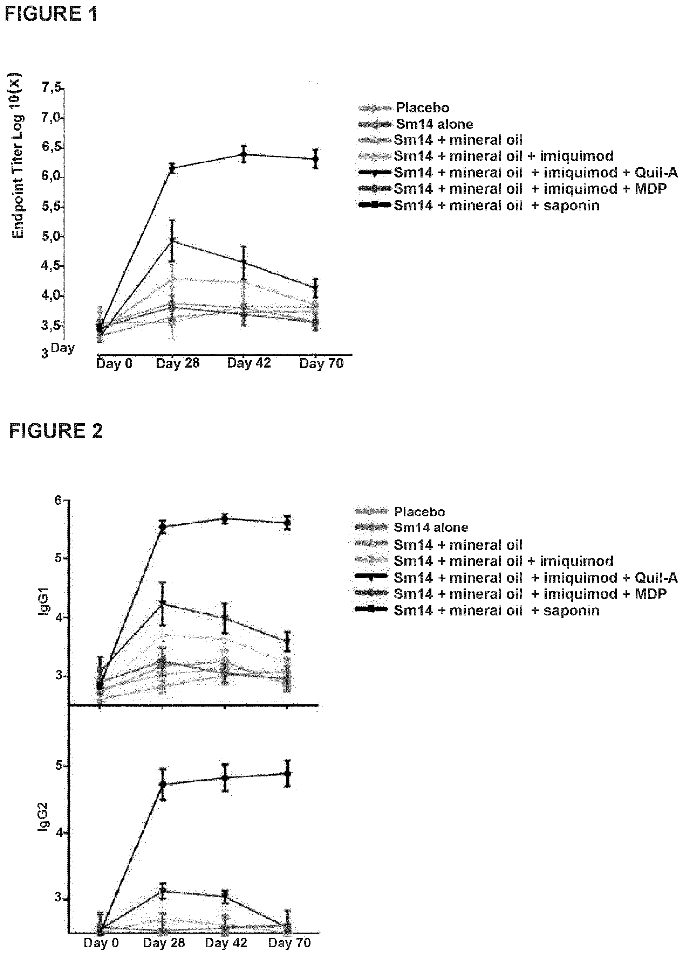

Figure 1 shows the results of the analysis of the total IgG antibody titre against the Sm14 protein. **Figure 1** shows the titration of total IgG antibodies in individual sera from animals immunized with different formulations. The dots show the mean of each group with its respective standard deviation.

Figure 2 shows the IgG1 and IgG2 antibody titers against the Sm14 protein. **Figure 2** shows the titration of IgG1 and IgG2 antibodies in individual sera from animals immunized with different formulations. The dots show the mean of each group with its respective standard deviation.

The results shown in Figures 1 and 2 show that the Sm14 protein alone or in the presence of oil (W/O formulation) is unable to stimulate the immune response. The addition of adjuvants was therefore evaluated.

The addition of the adjuvant Imiquimod (TLR-7 agonist) increases the immune response in the formulation with 2 % oil (diamond) in Figures 1 and 2). The addition of Quil-A (purified saponin fraction) to this formulation has a synergistic effect, increasing the response of total IgG antibodies and IgG1 and IgG2 (inverted triangle) in Figures 1 and 2). On the other hand, the addition of MDP (NOD receptor agonist) cancels out the antibody response against the Sm14 protein in the oil formulation with Imiquimod (circle) in Figures 1 and 2).

The water-in-oil and saponin formulation showed a higher response (by two orders of magnitude) than the oil-in-water formulations, in terms of total antibody titer (rectangle) in Figure 1), and the IgG1 and IgG2 subtypes against the Sm14 protein (rectangle) in Figure 2). The fact that there was a high response to these two IgG subtypes indicates that this formulation is capable of inducing both humoral and cellular immune responses.

In the composition of the present invention, either Quil-A (purified component of saponin) or saponin itself can be used.

This formulation releases the antigen slowly, making the response last longer than the other formulations (rectangle) in Figures 1 and 2). Oil formulations, which are commonly used in veterinary vaccines, increase the intensity and duration of the immune response [Fukanoki et al., 2000; Jansen et al., 2005,2006; Miles et al., 2005].

Therefore the water-in-oil and Quil-A/saponin formulation is used in the veterinary vaccine based on the Sm14 and FhFABP-3 proteins of the present invention.

### EXAMPLE 2 - Comparison of water-in-oil oil formulations

Two oil formulations (water-in-oil) were assessed using Marcol 52 as the oil phase and saponin as the adjuvant. The effect of the surfactant compounds used for the emulsion, Span 80 +Tween 80 (Formulation A in Table 2) was compared with the broad-chain surfactant Cetyl PEG/PPG-10/1 Dimethicone. The aqueous antigen formulation with saponin and Thimerosal (Formulation C in Table 2) was used as a control.

**Table 2: Composition of the 2 ml dose of A/O formulations for the vaccine containing the Sm14 antigen**

| **Phase** | **Compound** | **Formulation** | | |
|---|---|---|---|---|
| | | **A** | **B** | **C** |
| Aqueous phase (40 %) | Antigen (Sm14) | 80 ug | 80 ug | 80 ug |
| | Saponin | 1 mg | 1 mg | 1 mg |
| | Thimerosal | 0.01 % | 0.01 % | 0.01 % |
| Oil phase (60 %) | Surfactant | Span80 64 mg Tween-80 18 mg | Cetyl PEG/PPG-10/1 Dimethicone 20 mg | - |
| | Marcol 52 | 1.2 ml | 1.2 ml | - |

The emulsions of Formulas A and B, in a volume of 1 liter, were prepared under aseptic conditions, passing the mixture three times (three passes) through the high-speed Silverson Homogenizer, at 9000 rpm, keeping the temperature between 15 and 37 °C. The material was distributed into 50 ml plastic vaccine vials (primary packaging), capped, and recapped by hand.

Formulation C was obtained by simply mixing the components in an aqueous solution.

Groups of 10 adult animals (sheep) were immunized at 0 and 28 days with 2 ml doses of Formulas A, B and C. A fourth group remained unvaccinated (control group). The animals' serum was collected every 14 days for analysis.

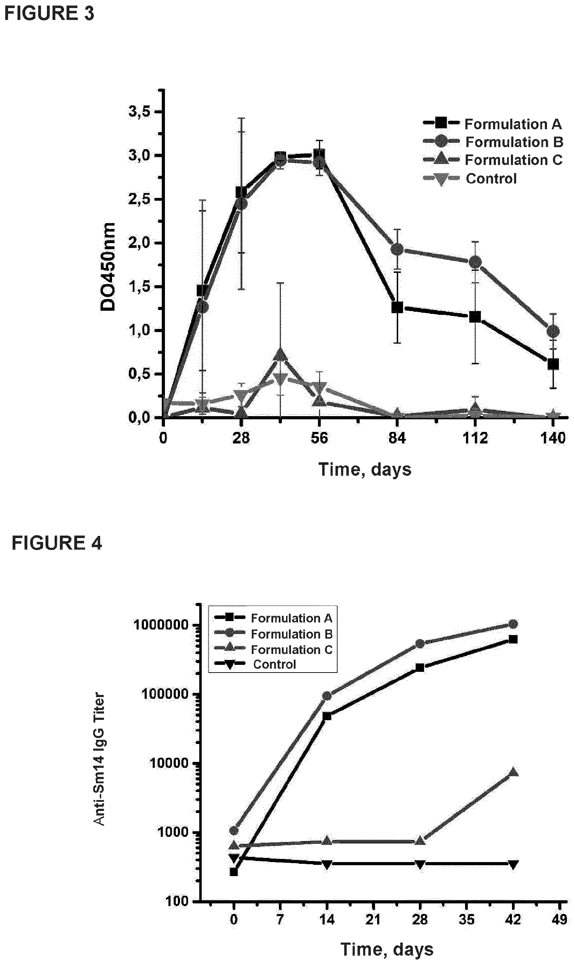

In **Figure 3** the average of the ELISA readings of IgG anti-Sm14 antibodies in individual sera from the experiment comparing oil formulations in sheep (dilution 1/2000) is shown. The dots show the mean of each group with its respective standard deviation.

At dilution 1/2000, the ELISA values at 42 and 56 days for Formulations A and B were at the upper limit of detection. In order to differentiate the responses of these two formulations, we titrated the IgG antibodies in the pool of sera from these groups between 0 and 42 days.

In **Figure 4** the titration of pools of sera from the experimental groups for anti-Sm14 IgG antibodies is shown.

The titration analysis showed that Formulation B gave a superior response to Formulation A. Both Formulation A and B gave a far superior response to the aqueous Formulation with saponin.

Western Blot experiments were conducted to study the specificity of the pools of sera from the group immunized with Formulation B.

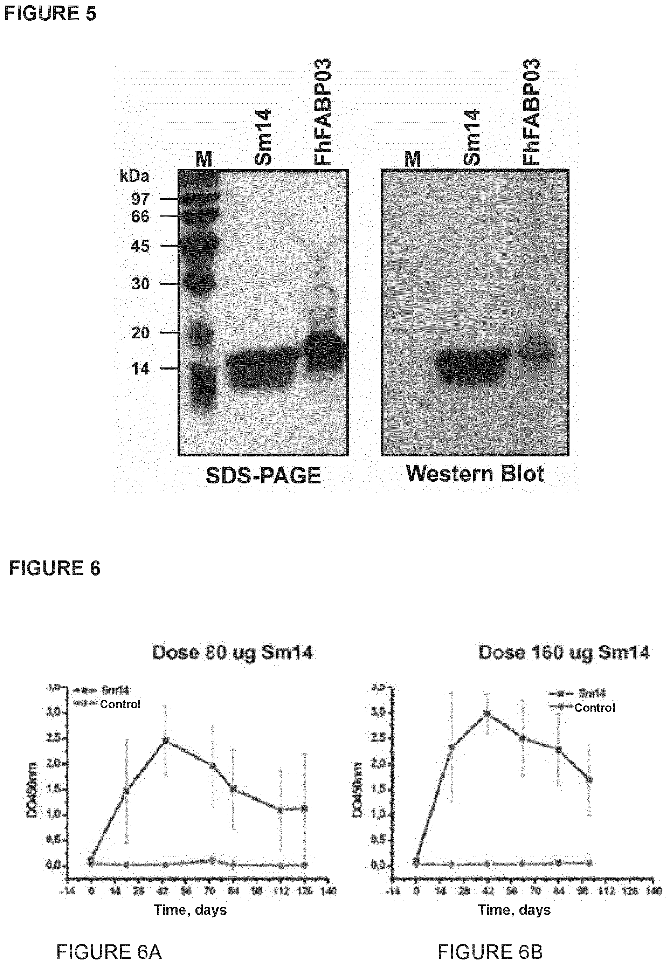

**Figure 5** shows the Western Blotting analysis of the recognition of the Sm14 and FhFABP-3 protein from *Fasciola hepatica* by the pool of sera from animals immunized with Formulation B, 42 days after immunization (1/10000 dilution).

The pool of sera from animals immunized with Formulation B at 42 days was able to specifically recognize the Sm14 protein at a dilution of 1/10000. This serum also recognizes the FhFABP-3 protein, homologous to *Fasciola hepatica,* purified under the same conditions as Sm14.

This result is important because it shows that immunization with the Sm14 protein can confer protection against infection by *F. hepatica* and *vice* versa, immunization with FhFABP-3 can confer protection against infection by S. *mansoni,* as reported by López-Abán et al (2016) (López-Abán, J., Rojas-Caraballo, J. et al. Protection against Schistosoma mansoni infection using a Fasciola hepatica-derived fatty acid binding protein from different delivery systems. Parasites Vectors 9, 216 (2016).

### EXAMPLE 3 - Dose-response study in bovine immunization

Fasciolosis is especially important for the cattle industry, which is why the immunization of adult cattle with Formulation B was studied.

Initially, 10 adult animals were immunized with the 80 ug dose of antigen in 2 ml that had demonstrated a high and homogeneous antibody response in sheep. A group of non-immunized animals was used as a control. **Figure 6A** shows the result of this immunization. Immunization of cattle with the same dosage used in sheep proved to be lower and more heterogeneous, with some animals responding significantly below average.

In order to improve the immune response, the volume of the dose was doubled to apply 160 ug of antigen per immunization. Groups of 10 animals were immunized with 4 ml of Formulation B (Sm14), while another 10 were left unvaccinated (control).

**Figure 6B** shows that increasing the dose of Sm14 protein in Formulation B from 2 ml (80 ug of Sm14) to 4 ml (160 ug of Sm14) resulted in a more intense and homogeneous immune response in cattle.

In **Figure 6** the average ELISA readings of anti-Sm14 IgG antibodies in individual sera from cattle immunized with 80 ug (Fig. 6A) and 160 ug (Fig. 6B) of the Sm14 antigen (dilution 1/800). The dots show the mean of each group with its respective standard deviation.

The result is that the same formulation of Sm14 can be used for both sheep and cattle, with a change in the volume to be applied: 2 ml (80 ug of Sm14) for sheep and 4 ml (160 ug of Sm14) for cattle.

### EXAMPLE 4 - Stability study of the water-in-oil formulation.

### A.- Emulsion breakdown

In order to study the stability of the antigen in the water-in-oil formulation, it is necessary to recover the protein from the aqueous phase, which is found forming droplets within an oily medium. This is done by breaking the emulsion using the following protocol:
- Separating a 1 mL aliquot of the vaccine, previously homogenized vigorously, into a 2 ml Eppendorf tube.
- Adding 1 mL of organic solvent (chloroform).
- Shaking it vigorously in a vortex.
- Incubating it for 16 hours at 4 °C.
- Centrifugating it at 12,000 rpm for 30 minutes in a microcentrifuge.
- Transferring the upper (aqueous) phase to a new Eppendorf tube.
- Adding Trichloroacetic Acid (TCA) at a final concentration of 10 % and incubate at -20 °C to precipitate the protein.
- Centrifugating at 12,000 rpm for 30 minutes in a microcentrifuge.
- Washing with 0.5 ml of ice-cold acetone twice, centrifugating at the same time and speed.
- Allowing the acetone to evaporate and resuspending the protein in water.

The protein thus obtained can be analyzed by SDS-PAGE and Western Blot.

### B.- Stability for 20 months at room temperature

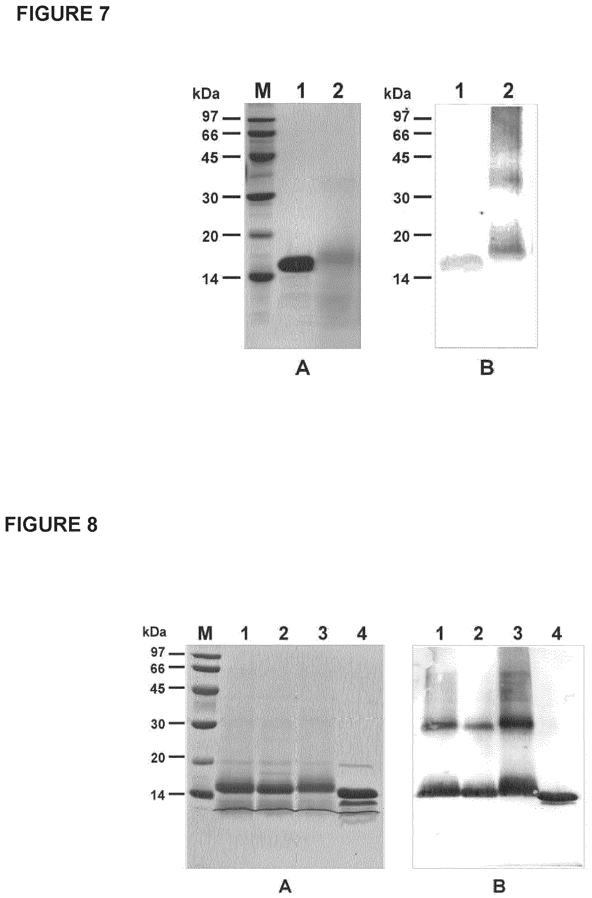

A sample vial (primary packaging) with Formulation B was kept at room temperature (around 22°C) for 20 months without the emulsion breakdown during storage in this condition. After the emulsion broke, the antigen present in this vaccine was analyzed. Figure 7 shows the result of the analysis.

**Figure 7** is the SDS-PAGE and Western Blot analysis of the Sm14 protein of Formulation B after 20 months of storage at room temperature, wherein **A**.- SDS-PAGE; **B**.- Western Blot. **1**.- Control protein, **2**.- Protein extracted from emulsion breakdown.

After 20 months of storage at room temperature (around 22 °C) it was still possible to observe a band corresponding to the Sm14 protein that was successfully recognized by the anti-Sm14 serum, without degradation, but with the formation of multimers.

### C.- Accelerated stability

An accelerated stability study was conducted with Formulation B, keeping the vaccine formulated and aliquoted in the primary packaging (50 ml plastic bottles) at different temperatures for a period of one month:
- Refrigerator (4 °C - 8 °C)
- Room temperature (22 °C), and
- In the stove at 37 °C

After this period, samples from each of the storage conditions were subjected to emulsion breakdown and analysis by SDS-PAGE and Western Blot.

**Figure 8** shows Sm14 protein analysis in Formulation B stored for one month at different temperatures, wherein **A**.- SDS-PAGE; **B**.- Western Blot. **1**.- Vaccine stored at room temperature, **2**.- Vaccine stored at 4 °C, **3**.- Vaccine stored at 37 °C, **4**.- Control.

**Figure 8** indicates that there were no differences in stability at the three temperatures studied after one month's storage. Again, there was no degradation of the protein, since this phenomenon would show bands of lower molecular mass being recognized by the anti-Sm14 antibodies. However, the formation of multimers was observed, which may have been generated by the treatment of the emulsion breakdown with the organic solvent or trichloroacetic acid.

### D.- Stability of immune response induction

To assess whether the formulation remains immunogenic after refrigeration storage for one year, sheep immunization was compared at 0 and 12 months after formulation.

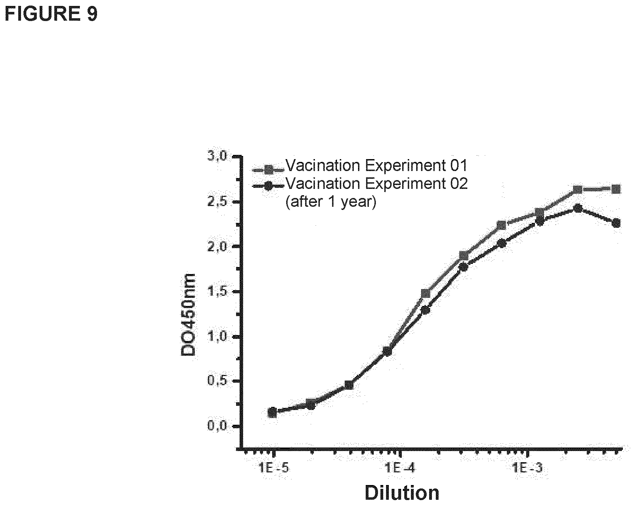

**Figure 9** shows the titration of pools of sera for IgG anti-Sm14 antibodies at 42 days from two immunization experiments conducted with the same batch of vaccine, one year apart. Adult sheep were used in both immunization experiments.

In **Figure 9** it can be seen that the titration curves of the sera from two immunization experiments, conducted one year apart, overlap, with minor variation in the lower dilutions, indicating that the immunogenicity of the formulation has remained stable for one year.

It was observed that the water-in-oil emulsion of Formulation B remains stable for at least two years under refrigeration. Analysis of the antigen recovered by emulsion breakdown indicates that the Sm14 protein also remains stable in this condition.

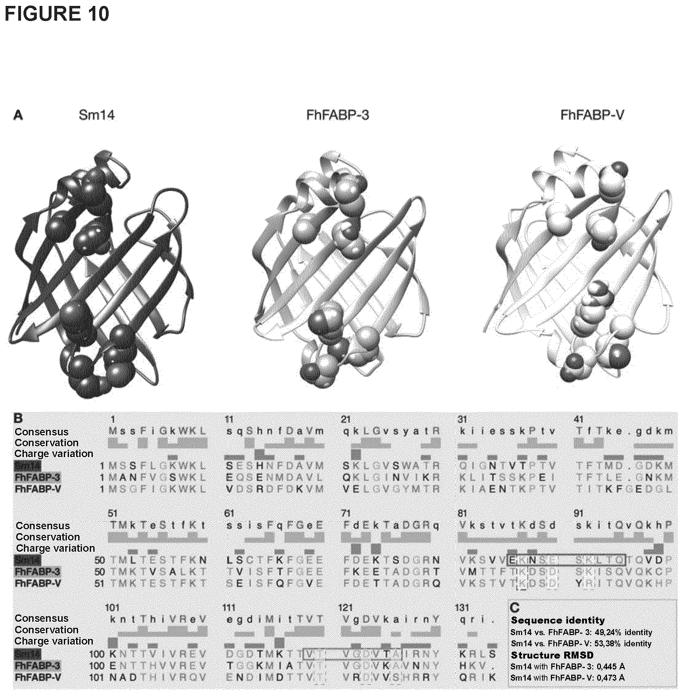

**Figure 10** aims to demonstrate that there is high structural conservation including immunogenic epitopes between the Sm14 and the FABPs from *Fasciola hepatica.* **Figure 10A** is a cartoon representation of the Sm14 protein solved by crystallography (PDB ID 1VYF, in black, left) and models of the structures of FhFABP-3 (gray, center) and FhFABP-V (white, right) built by the I-TASSER server [J Yang, Y Zhang. I-TASSER server: new development for protein structure and function predictions, Nucleic Acids Research, 43: W174 - W181, 2015]. Highlight in sphere representation and colored in CPKfor residues conserved between the structures of immunogenic peptides 1.1 and 2.1 [Vilar MM, Barrientos F, Almeida M, et al., An experimental bivalent peptide vaccine against schistosomiasis and fascioliasis. Vaccine 2003; 22(1): 137 - 44. Zafra R, Buffoni L, Pérez-Écija RA, et al., Study of the local immune response to Fasciola hepatica in the liver and hepatic lymph nodes of goats immunized with a peptide of the Sm 14 antigen. Research in Veterinary Science 2009; 87(2): 226 - 32. Zafra R, Buffoni L, Martinez-Moreno A, Pérez-Écija A, Martinez-Moreno FJ, Pérez J. A Study of the Liver of Goats Immunized with a Synthetic Peptide of the Sm14 Antigen and Challenged with Fasciola hepatica. Journal of Comparative Pathology 2008; 139(4): 169 - 76]. **Figure 10B** shows the structural alignment (Match-Align) performed with the UCSF Chimera software [Pettersen EF, Goddard TD, Huang CC, et al. UCSF Chimera - a visualization system for exploratory research and analysis. J Comput Chem 2004; 25(13): 1605 - 12], showing consensus sequence, qualitative index of sequence conservation and amino acid charge variation. Immunogenic peptides 1.1 (highlighted in blue) and 2.1 (highlighted in red) with residues in yellow with sphere representation in (A). **Figure 10C** shows the percentage of identity between the sequences and RMSD between the structures of Sm14 and the two FABPs from *Fasciola hepatica.* The identity between the sequences is around 50 %, but shows great structural conservation, with an average squared deviation below 0.5 Å calculated by the Match-Align algorithm in the UCSF Chimera software.

Optionally, the composition of the present invention is administered parenterally. Still optionally, the composition of the present invention is administered by parenteral injection. Still optionally, said composition is administered subcutaneously. Still optionally, the composition of the present invention is administered by subcutaneous injection. Optionally, the composition of the present invention is administered orally, including sublingual, sublabial and/or buccal and/or nasal.

From the above description, we show that the invention described herein shows that the Sm14 protein and the FABPs from *Fasciola hepatica* are able to provide a homogeneous and long-lasting immune response against helminths.

## Claims

1. Veterinary vaccine composition against helminths **characterized by** comprising:
(a) an aqueous phase, corresponding to 40 % (volume/volume) of the dose, which can be 2 to 4 ml, containing:
- from 50 ug to 200 ug of antigen defined by any of the sequences SEQ ID NO: 1, SEQ ID NO: 2, SEQ ID NO: 3, SEQ ID NO: 4, SEQ ID NO: 5, SEQ ID NO: 6 or SEQ ID NO: 7;
- 0,5 mg to 1 mg of saponin or Quil-A;
- 50 mM of Tris-HCl buffer; and
- 0,01 % of thimerosal,
(b) an oil phase containing:
- Marcol 52^{™} oil in an amount corresponding to 60 % (volume/volume) of the dose;
- 10 to 40 mg of a surfactant selected from Cetyl PEG/PPG-10/1 Dimethicone, Span 80 or Tween 80.

2. Composition according to the claim 1 **characterized by** the surfactant used being preferably Cetyl PEG/PPG-10/1 Dimethicone.

3. Composition according to the claim 1 **characterized by** the surfactants being used in a quantity of 20 mg of Cetyl PEG/PPG-10/1 Dimethicone or 64 mg of Span 80 or 18 mg of Tween 80, for a dose of 2 ml volume.

4. Composition according to the claim 1 **characterized by** the antigen being preferably selected from among the sequences SEQ ID NO: 5, SEQ ID NO: 6 and SEQ ID NO: 7.

5. Composition according to the claim 1 **characterized by** said composition being applied against infection caused by *Fasciola hepatica.*

6. Composition according to the claim 1 **characterized by** said composition being used in animals selected from cattle, sheep and goats.

7. Composition according to the claim 1 **characterized by** said composition being administered parenterally, by parenteral injection, subcutaneously, by subcutaneous injection, or orally, including sublingual, sublabial, and/or buccal and/or nasal.

8. Method for the treatment and prevention of infection caused by helminths **characterized by** said method employing the vaccine composition as defined in any one of claims 1 to 7.

9. Use of any of the proteins defined by the sequences SEQ ID NO: 1, SEQ ID NO: 2, SEQ ID NO: 3, SEQ ID NO: 4, SEQ ID NO: 5, SEQ ID NO: 6 or SEQ ID NO: 7 in a helminth vaccine composition.

10. Use according to the claim 9 **characterized by** being for use in a vaccine against infection caused by *Fasciola hepatica.*
